# EUROPEAN PATENT SPECIFICATION

(11) **EP 1 145 012 B1**
(45) Date of publication and mention of the grant of the patent: **18.08.2004**
(21) Application number: 00906904.8
(22) Date of filing: 12.01.2000
(51) Int. Cl.: G01N 33/68

(54) **METHODS AND KITS FOR SEQUENCING POLYPEPTIDES**
METHODEN UND KITS ZUM SEQUENZIEREN VON POLYPEPTIDEN
PROCEDES PERMETTANT DE SEQUENCER DES POLYPEPTIDES ET KITS A CET EFFET

(30) Priority: 20.01.1999 US 116502 P; 29.09.1999 US 156677 P
(43) Date of publication of application: 17.10.2001
(73) Proprietor: The Procter & Gamble Company, Cincinnati, Ohio 45202 (US)
(72) Inventor: KEOUGH, Thomas, Woods, Cincinnati, OH 45242 (US); YOUNGQUIST, Robert, Scott, Mason, OH 45040 (US)
(74) Representative: Veronese, Pancrazio
(86) International application number: PCT/US2000/000790
(87) International publication number: WO 2000/043792

(56) References cited:
- COX K A ET AL: "Role of the Site of Protonation in the Low-Energy Decompositions of Gas-Phase Peptide Ions" JOURNAL OF THE AMERICAN SOCIETY FOR MASS SPECTROMETRY,US,ELSEVIER SCIENCE INC, vol. 7, no. 6, 1 June 1996 (1996-06-01), pages 522-531, XP004052038 ISSN: 1044-0305 cited in the application
- NAKANISHI T ET AL: "Electrospray Ionization--Tandem Mass Spectrometry Analysis of Peptides Derived by Enzymatic Digestion of Oxidized Globin Subunits: An Improved Method to Determine Amino Acid Substitution in the Hemoglobin 'Core'" JOURNAL OF THE AMERICAN SOCIETY FOR MASS SPECTROMETRY,US,ELSEVIER SCIENCE INC, vol. 7, no. 10, 1 October 1996 (1996-10-01), pages 1040-1049, XP004070144 ISSN: 1044-0305
- ROTH K.D.W., HUANG Z.-H., SADAGOPAN N., WATSON J.T.: "Charge derivatization of peptides for analysis by mass spectrometry" MASS SPECTROM. REV., vol. 17, 1998, page 255 XP000922702
- HUANG Z.-H., WU J., ROTH K.D.W., YANG Y., GAGE D.A., WATSON J.T.: "A picomole-scale method for charge derivatization of peptides for sequence analysis by mass spectrometry" ANALYTICAL CHEMISTRY, vol. 69, 1997, pages 137-144, XP002141708
- KEOUGH, T.; YOUNGQUIST, R. S.; LACEY, M. P.: "A method for high-sensitivity peptide sequencing using postsource decay matrix-assisted laser desorption ionization mass spectrometry" PROC. NATL. ACAD. SCI. U. S. A., vol. 96, June 1999 (1999-06), pages 7131-7136, XP002141709
- DATABASE CHEMICAL ABSTRACTS [Online] American Chemical Society (via STN, Karlsruhe); Accession Number 2000:391605 (CAPLUS), BAUER, MARK D.; SUN, YIPING; KEOUGH, THOMAS ; LACEY, MARTIN P.: "Sequencing of sulfonic acid derivatized peptides by electrospray mass spectrometry" XP002141711 & RAPID COMMUN. MASS SPECTROM. , vol. 14, no. 10, 2000, pages 924-929,
- HUANG ZHI-HENG; SHEN TUNLI; WU JIANG; GAGE DOUGLAS A; WATSON J THROCK: "Protein sequencing by matrix-assisted laser desorption ionization-postsource decay-mass spectrometry analysis of the N-Tris(2,4,6-trimethoxyphenyl)phosphine-ac etylated tryptic digests" ANALYTICAL BIOCHEMISTRY, vol. 268, 15 March 1999 (1999-03-15), pages 305-317, XP002141710
- CHEN X ET AL: "Isotope Edited Product Ion Assignment by alpha-N Labeling of Peptides with [H3(50%)]2,4-Dinitrofluorobenzene" JOURNAL OF THE AMERICAN SOCIETY FOR MASS SPECTROMETRY,US,ELSEVIER SCIENCE INC., NEW YORK, NY, vol. 10, no. 5, May 1999 (1999-05), pages 448-452, XP004164485 ISSN: 1044-0305

## Description

### FIELD OF THE INVENTION

The present invention relates to methods and kits which enable polypeptide sequencing using mass spectrometric techniques. The methods and kits are particularly useful for identifying high molecular weight polypeptides for use in, for example, biological, pharmaceutical, personal cleansing. and fabric cleaning fields.

### BACKGROUND OF THE INVENTION

Recently, matrix-assisted laser desorption ionization (MALDI) mass spectrometry and electrospray ionization were developed for high-sensitivity peptide and polypeptide sequencing applications. See, e.g., Spengler et al., "Peptide Sequencing by Matrix-assisted Laser-desorption Mass Spectrometry", Rapid Communications in Mass Spectrometry, Vol. 6, pp. 105 - 108 (1992); Spengler et al., "Fundamental Aspects of Postsource Decay in Matrix-Assisted Laser Desorption Mass Spectrometry", Journal of Physical Chemistry, Vol. 96, pp. 9678 - 9684 (1992); Kaufmann et al., "Mass Spectrometric Sequencing of Linear Peptides by Product-ion Analysis in a Reflectron Time-of-flight Mass Spectrometer Using Matrix-assisted Laser Desorption Ionization", Rapid Communications in Mass Spectrometry, Vol. 7, pp. 902 - 910 (1993); Kaufmann et al., "Sequencing of Peptides in a Time-of-flight Mass Spectrometer: Evaluation of Postsource Decay Following Matrix-assisted Laser Desorption Ionisation (MALDI), International Journal of Mass Spectrometry and Ion Processes, Vol. 131, pp. 355 - 385 (1994): Kaufmann et al., "Post-source Decay and Delayed Extraction in Matrix-assisted Laser Desorption/Ionization-Reflectron Time-of-Flight Mass Spectrometry", Rapid Communications in Mass Spectrometry, Vol. 10, pp. 1199 - 1208 (1996); and Spengler, "Post-source Decay Analysis in Matrix-assisted Laser Desorption/Ionization Mass Spectrometry of Biomolecules", Journal of Mass Spectrometry, Vol. 32, pp. 1019 - 1036 (1997); Carr et al., "Integration of Mass Spectrometry in Analytical Biotechnology", Analytical Chemistry, Vol. 63, pp. 2802 - 2824, (1991); Yates III et al., "Mining Genomes With MS", Analytical Chemistry, Vol. 68, pp. 534A - 540A, (1996); Morris et al., "High Sensitivity Collisionally Activated Decomposition Tandem Mass Spectrometry on a Novel Quadrupole/Orthagonal Acceleration Time-of-Flight Mass Spectrometer", Rapid Communications in Mass Spectrometry, Vol. 10, 889 - 896, (1996).

MALDI offers several advantages in the field of mass spectrometry. For example, MALDI provides higher sensitivity than conventional electrospray triple quadrupole equipment. When used in combination with time-of-flight mass analyzers. MALDI is also applicable to higher mass peptides than can be analyzed with triple quadrupole equipment. MALDI is also useful for analyzing complex mixtures with minimal sample purification. Electrospray ionization, on the other hand, is readily interfaced to powerful separation techniques including liquid chromatography (LC) and various forms of capillary electrophoresis (CE). Highly automated analyses are possible when using LC and CE as the sample purification and introduction devices.

However, current MALDI and, to a lesser extent, electrospray ionization mass spectrometric methods fail to adequately offer predictable tandem mass spectrometry fragmentation patterns. For example, multiple ion series (including a-ions, b-ions, and y-ions) are typically observed, resulting in MALDI post-source decay spectra that are too complex for efficient interpretation and sequencing. Multiple ion series (b- and y-ions), plus internal fragments and both singly and multiply charged ions are formed from multiply charged precursor ions generated by electrospray ionization. and the resulting tandem mass spectra are often difficult to interpret de novo. Accordingly, problems with fragmentation have limited the ability to rapidly sequence polypeptides using mass spectrometry. As a result, mass spectrometry, and particularly MALDI mass spectrometry, is of limited value in this area.

Several research groups have attempted to improve the utility of mass spectrometry in the field of polypeptide sequencing through the use of chemical derivatization techniques. Such techniques have been utilized to promote and direct fragmentation in the MSMS spectra of peptides with the goal of increasing sensitivity and decreasing the complexity of the resulting spectra. Most of these known techniques provide cationic derivatives. See, e.g., Kidwell et al., "Sequencing of Peptides by Secondary Ion Mass Spectrometry", Journal of the American Chemical Society, Vol. 106, pp. 2219 - 2220 (1984) (derivatization with a quaternary ammonium group, analysis using the static SIMS ionization method (prior to development of both MALDI and electrospray ionization)). Application of such techniques using MALDI and electrospray ionization with low-energy collisional activation have not proven generally effective.

More recently, researchers have utilized other derivatization techniques in an effort to enhance peptide / polypeptide sequencing using mass spectrometric methods. For example, it has been shown that oxidation of cysteine residues present in tryptic peptides (peptides produced via digestion with trypsin) may improve fragmentation using electrospray tandem mass spectrometry. See, e.g., Gaskell et al., "Role of the Site of Protonation in the Low-Energy Decompositions of Gas-Phase Peptide Ions", Journal of the American Society of Mass Spectrometry, Vol. 7, pp. 522 - 531 (1996) and Gaskell et al., "Influence of Cysteine to Cysteic Acid Oxidation on the Collision-Activated Decomposition of Protonated Peptides: Evidence for Intraionic Interactions", Journal of the American Society of Mass Spectrometry, Vol. 3, pp. 337 - 344 (1992). Specifically, y-ion fragmentation was promoted. However, this technique has several limitations. For example, the technique was not extended to MALDI methods. The technique is also limited to the analysis of those polypeptides having cysteine residues occurring in the sequence to be analyzed. Indeed, cysteine occurs rather rarely in naturally occurring polypeptides, placing a severe limitation on the utility of this technique.

Accordingly, there is a need to provide a mass spectrometric method of sequencing polypeptides that is simple, efficient, and widely applicable to wild-type and variant polypeptides. The present inventors herein provide a method for high-sensitivity polypeptide sequencing using mass spectrometric techniques. The present inventors have discovered that polypeptides and peptides thereof derivatized with relatively strong acid groups will provide y-ion fragmentation nearly exclusively. resulting in spectra which are easily interpreted de novo. The present invention is also related to kits which are used to conveniently enable performance of the present method.

### SUMMARY OF THE INVENTION

The present invention relates to mass spectrometric methods and kits which are particularly useful for sequencing polypeptides. The methods involve determining the amino acid sequence of a polypeptide, the steps comprising:
(a) derivatizing the N-terminus of the polypeptide or the N-termini of one or more peptides of the polypeptide with one or more acidic moieties having a pKas of less than about 2 when coupled with the polypeptide or peptides, to provide one or more derivatized analytes;
(b) analyzing one or more derivatized analytes using a mass spectrometric technique to provide a fragmentation pattern; and
(c) interpreting the fragmentation pattern.

The kits of the present invention comprise one or more acidic moiety reagents which provide acidic moieties having pKas of less than about 2 when coupled with a polypeptide; and means for derivatizing the N-terminus of the polypeptide or the N-termini of one or more peptides of the polypeptide with one or more acidic moiety reagents. The present kits are particularly useful in conjunction with performance of the methods.

### DETAILED DESCRIPTION OF THE INVENTION

The methods and kits of the present invention are useful for sequencing polypeptides including, for example, wild-type, variant, and / or synthetic polypeptides. The methods and kits are particularly useful for identifying high molecular weight polypeptides for use in, for example, biological, pharmaceutical, personal cleansing, and fabric cleaning fields.

The present methods and kits have widespread utility. Applications include, but are not limited to: facilitation of biological studies requiring rapid determination of peptide or polypeptide sequences; identification of post-translational modifications in proteins and for the identification of amino acid modifications in variant proteins such as those used in, for example, commercial laundry and cleansing products; aiding the design of oligonucleotide probes for gene cloning: rapid characterization of products formed in directed evolution studies; combinatorial chemistry and peptide library identification; and proteomics.

The present method involves addition of one or more relatively strong acid groups to the N-terminus of the polypeptide or one or more peptides formed through cleavage of the polypeptide prior to mass spectrometric analysis. Without intending to be limited by theory, it is believed that the resulting negatively charged derivative(s) facilitate low energy charge-site initiated fragmentation. This is especially effective wherein the C-termini of the polypeptide or peptides thereof are basic or hydrophobic, preferably basic residues. Again, without limitation by theory, it is believed that wherein a basic residue is protonated during mass spectrometric analysis, the relatively strong acid group will be deprotonated, which counter-balances the positive charge at the basic residue. An additional proton to ionize the derivative will be substantially "free" to randomly ionize the backbone amide groups of the polypeptide / peptide because the most basic residue is already protonated. Additionally, without limitation by theory, it is believed that wherein a second relatively strong acid group is incorporated into the polypeptide / peptide that both acid groups will be deprotonated, providing two substantially "free" protons to randomly ionize the backbone amide groups of the polypeptide / peptide.

Utilization of the present method provides significant increases in fragmentation efficiencies. Furthermore, increased fragment ion signal-to-noise ratios are observed for derivatized peptides relative to non-derivatized peptides having the same sequences. The resulting simple PSD MALDI and electrospray tandem mass spectra can be routinely interpreted de novo.

All percentages, ratios, and proportions used herein are by weight unless otherwise specified.

As used herein, abbreviations will be used to describe amino acids. Such abbreviations are described in Table 1 below. Further described in Table I are average amino acid residue masses which are useful for the practice of the present invention.

**Table I**

| Amino Acid | Three-letter Abbreviation | One-Letter Abbreviation | Average Amino Acid Residue Mass (Da) |
|---|---|---|---|
| Alanine | Ala | A | 71.1 |
| Arginine | Arg | R | 156.2 |
| Asparagine | Asn | N | 114.1 |
| Aspartic Acid | Asp | D | 115.1 |
| Cysteine | Cys | C | 103.1 |
| Glutamine | Gln | Q | 128.1 |
| Glutamic Acid | Glu | E | 129.1 |
| Glycine | Gly | G | 57.1 |
| Histidine | His | H | 137.1 |
| Isoleucine | Ile | I | 113.2 |
| Leucine | Leu | L | 113.2 |
| Lysine | Lys | K | 128.2 |
| Methionine | Met | M | 131.2 |
| Phenylalanine | Phe | F | 147.2 |
| Proline | Pro | P | 97.1 |
| Serine | Ser | S | 87.1 |
| Threonine | Thr | T | 101.1 |
| Tryptophan | Trp | W | 186.2 |
| Tyrosine | Tyr | Y | 163.2 |
| Valine | Val | V | 99.1 |

### Definitions

As used herein, the term "desorption ionization" refers to the transition of an analyte from the solid-phase to the gas-phase as ions.

As used herein, the term "desorption" refers to the departure of analyte from the surface and / or the entry of the analyte into a gaseous phase.

As used herein, "ionization" refers to the process of creating or retaining on an analyte an electrical charge equal to plus or minus one or more electron units.

As used herein, the term "MALDI" refers to matrix-assisted laser desorption ionization.

As used herein, the term "matrix" in reference to "MALDI" refers to small, acidic, light absorbing chemicals which may be mixed in solution with the polypeptide. of interest or peptides thereof, of interest in such a manner so that, upon drying on the sample stage, the crystalline matrix-embedded analytes can be successfully desorbed and ionized from the solid-phase into the gaseous or vapor-phase following laser irradiation. Alternatively, solutions of polypeptides, or peptides thereof, may be loaded onto appropriate matrices which are pre-dried on the sample stage. Non-limiting examples of suitable matrices include nicotinic acid, sinapinic acid. ferulic acid, caffeic acid, a-cyano-4-hydroxycinnamic acid, and a-cyano-4-hydroxycinnamic acid mixed with nitrocellulose.

As used herein, the term "electrospray ionization" refers to the process of producing ions from solution by electrostatically spraying the solution from a capillary electrode at high voltage with respect to a grounded counter electrode. The definition is intended to include both electrospray ionization and pneumatically assisted electrospray ionization, which is also referred to as ionspray. As used herein, the term "electrospray ionization" applies to all liquid flow rates and is intended to include microspray and nanospray experiments. Moreover, the definition is intended to apply to the analyses of peptides directly infused into the ion source without separation, and to the analysis of peptides or peptide mixtures that are separated prior to electrospray ionization. Suitable on-line separation methods include, but are not limited to, HPLC, capillary HPLC and capillary electrophoresis. Electrospray ionization experiments can be carried out with a variety of mass analyzers, including but not limited to, triple quadrupoles, ion traps, orthogonal-acceleration time-of-flight analyzers and Fourier Transform Ion Cyclotron Resonance instruments.

As used herein, the term "polypeptide" refers to a molecule having two or more amino acid residues. The method of the present invention is suitable for sequence identification of high mass polypeptides.

As used herein, the term "wild-type" refers to a polypeptide produced by unmutated organisms.

As used herein, the term "variant" refers to a polypeptide having an amino acid sequence which differs from that of the wild-type polypeptide.

### Methods of the Present Invention

The present method is useful for determining the amino acid sequence of a polypeptide. By using the phrase "determining the amino acid sequence", the present inventors do not intend to be limited to determining the entire sequence of a given polypeptide. Rather, by this phrase it is meant herein that a portion, portions, and / or the entire sequence is determined.

The present methods involve addition of one or more relatively strong acid groups to the N-terminus of a polypeptide or one or more peptides thereof to produce one or more derivatized analytes for mass spectrometric analysis. The polypeptide / peptides are then analyzed using a mass spectrometric technique to provide a fragmentation pattern. The resulting fragmentation pattern is interpreted, thereby allowing sequencing of the polypeptide.

The present inventors have discovered that the acidity of the derivatizing group(s) (acidic moiety) has a profound effect on the resulting mass spectra. Surprisingly, those acidic moieties having a pKa of less than about 2 when coupled with a polypeptide or peptide thereof will yield fragmentation patterns which are easily interpreted to provide the desired sequence information. The ordinarily skilled artisan is competent to measure the pKa values as described herein using standard methods known in the art. Non-limiting examples of such methods include, for example, titration and electrochemical methods. The preferred method of measuring pKa values is through titration.

The method of the present invention may be performed as follows:

### Derivatization of Polypeptide and / or Peptides of the Polypeptide

An important feature of the present invention is derivatization of a polypeptide or peptides of a polypeptide of interest with one or more a relatively strong acids, i.e., acidic moieties having pKas less than about 2, preferably less than about 0, and more preferably less than about -2, when coupled with a polypeptide or peptides of the polypeptide. By "peptides of a polypeptide" it is meant herein that the polypeptide is digested or otherwise cleaved (herein collectively referred to as "digestion" or the like) into two or more peptides. The resulting peptides are derivatized in accordance with the present method. By "when coupled" it is meant herein that the pKas of the acidic moieties is defined as measured after being covalently bonded with a polypeptide or peptide herein.

The polypeptide or peptides thereof may be produced by any means. For example, if necessary, the polypeptide of interest is isolated for analysis. Several procedures may be utilized for isolation including, for example, one-dimensional and two-dimensional gel electrophoresis. As another example, polypeptides may be synthesized through combinatorial chemistry methods well known in the art.

Digestion may occur through any number of methods, including in-gel or on a membrane, preferably in-gel. See, e.g., Shevchenko et al., "Mass Spectrometric Sequencing of Proteins from Silver-Stained Polyacrylamide Gels", Analytical Chemistry, Vol. 68, pp. 850 - 858 (1996). However, it is possible to digest the polypeptide either enzymatically or chemically, preferably enzymatically. It is most preferable to utilize a digestion procedure which yields a basic or hydrophobic residue, most preferably basic, at or near the C-terminus of the resulting peptides. By "at" it is meant herein that the basic or hydrophobic residue is the C-terminal residue of the peptide. By "near" it is meant herein that the basic or hydrophobic residue is preferably within about 40 amino acid residues from the C-terminus of the peptide, more preferably within about 30 residues, even more preferably about 20 residues. and most preferably within 10 amino acid residues from the C-terminus of the peptide.

While many methods may be utilized for this procedure, it is preferred to enzymatically digest the polypeptide using, for example, trypsin, endoproteinase Lys C, endoproteinase Arg C, or chymotrypsin, preferably, trypsin, endoproteinase Lys C, or endoproteinase Arg C. and most preferably trypsin. Trypsin, endoproteinase Lys C, and endoproteinase Arg C are preferable because the resulting peptides of the polypeptide will typically terminate at the C-terminus with an arginine or lysine residue (basic residues), with the exception, of course, of the original C-terminus of the polypeptide. Other enzymes are also suitable, especially if basic residues occur at or near the C-terminus of the resulting peptides. Chymotrypsin is also preferred for digestion, which typically cleaves at hydrophobic amino acid residues. Chemical digestion is also useful. For example, digestion with cyanogen bromide is useful.

The method of the present invention may be adapted according to that described in Patterson et al., U.S. Patent No. 5,821,063, assigned to PerSeptive Biosystems, Inc., issued October 13. 1998, particularly with respect to the digestion techniques described therein. For example, a plurality of samples having different ratios of agent to polypeptide may be utilized and derivatized according to the present invention.

However, digestion is not always necessary, particularly when sequencing (but certainly not limited to) small polypeptides. As used herein, "small" polypeptides include those having preferably less than about fifty amino acid residues, more preferably less than about forty residues. even more preferably less than about thirty residues, still more preferably less than about twenty residues, and most preferably less than about ten amino acid residues.

For example, polypeptides may be characterized which are synthesized by well-known means, including combinatorial chemistry methods (a "synthetic polypeptide"). In this instance, it is most preferable to synthesize a polypeptide having basic or hydrophobic residue, preferably basic (most preferably arginine or lysine), at or near the C-terminus of the resulting polypeptide.

The polypeptide (if the polypeptide is sufficiently "small" as defined herein above) or the peptides of the polypeptide are derivatized with one or more acidic moieties having pKas of less than about 2, preferably less than about 0, and most preferably less than about -2 (when coupled with the polypeptide or peptides) to provide a derivatized analyte. The acidic moieties of the derivatized analyte are prepared by coupling with an acidic moiety reagent. The acidic moiety reagent is not limited, provided an acidic moiety on the polypeptide or peptides thereof results having the herein described pKa. Non-limiting examples of acidic moiety reagents which may be utilized for coupling include, for example, dithiobis(sulfosuccinimidylpropionate), S-acetylmercaptosuccinic anhydride, 2-iminothiolane (which may also be referred to as Traut's reagent), dithiodiglycolic anhydride tetrafluorosuccinic anhydride. hexafluoroglutaric anhydride. sulfosuccinic anhydride, 2-sulfobenzoic acid cyclic anhydride, chlorosulfonylacetyl chloride, and 1,3-propane sultone. Use of reagents such as S-acetylmercaptosuccinic anhydride, 2-iminothiolane, and dithiodiglycolic anhydride require oxidation after derivatization with the peptide to produce the acidic moiety. Acidic moiety reagents not requiring the oxidation step include, for example, tetrafluorosuccinic anhydride, hexafluoroglutaric anhydride, sulfosuccinic anhydride, 2-sulfobenzoic acid cyclic anhydride and chlorosulfonylacetyl chloride. These reagents are often preferred. due to more efficient synthesis and / or lack of complicating oxidation of labile residues in the polypeptide or peptides thereof. Coupling an acidic moiety reagent to the N-terminus of a cysteine-containing peptide, followed by oxidation of the cysteine sulfhydryl group to cysteic acid, is one means to produce peptides containing two acidic moieties (sulfonic acids).

The acidic moieties are most preferably a sulfonic acid. Among these, the more preferred acidic moieties include 2-sulfoacetyl moiety, 3-sulfopropionyl moiety, and 2-sulfobenzoyl moiety.

The use of disulfonic acid derivatives is also preferred. Use of the disulfonic acid derivatives preferably results in both sulfonic acids groups near the N-terminus of the peptide. For example, coupling an acidic moiety reagent to the N-terminus of a cysteine-containing peptide, followed by oxidation of the cysteine sulfhydryl group to cysteic acid is one means to produce peptides containing two acidic moieties (sulfonic acids).

The ordinarily skilled artisan will have the ability to perform the relatively simple coupling procedures required by the present invention. For convenience, however, non-limiting examples of derivatization of polypeptides, or peptides of the polypeptide of interest, are illustrated as follows:

### Example 1

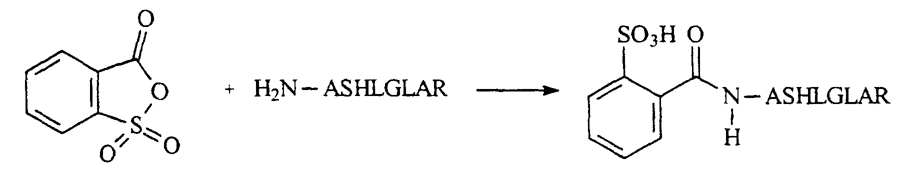

2-Sulfobenzoic acid cyclic anhydride (commercially available from Aldrich Chemical Co., Milwaukee, WI) is prepared at a concentration of 0.1 M in dry tetrahydrofuran prior to use. The polypeptide ASHLGLAR (1 nmol, commercially available from Sigma Chemical Co., St. Louis MO) (SEQ ID NO: 1) is diluted into 20 µL of 0.05 M trimethylamine. The 2-sulfobenzoic acid cyclic anhydride solution (2 µL) is added and the reaction mixture is vortexed for 30 seconds. The reaction proceeds for approximately 2 minutes at room temperature prior to dilution of the resulting derivatized analyte and mass spectral analysis. The concentration of the acidic moiety reagent is decreased by a factor of as much as 100 when derivatizing smaller quantities.

### Example 2

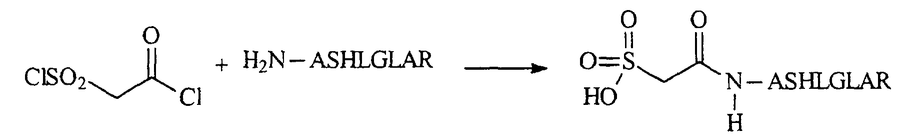

ASHLGLAR (1 nmol, commercially available from Sigma Chemical Co., St. Louis MO) (SEQ ID NO: 1) is mixed with 2 µL of 0.02 M sulfoacetic acid, which is formed by mixing 2 µL of neat chlorosulfonylacetyl chloride (commercially available from Aldrich Chemical Co., Milwaukee, WI) with 500 µL of water. The mixture is dried and then reconstituted in 20 µL of tetrahydrofuran:diisopropylethyl amine (4:1 v:v). 0.1 M chlorosulfonylacetyl chloride (2 µL, commercially available from Aldrich Chemical Co., Milwaukee, WI) in dry tetrahydrofuran is added and the mixture is vortexed for 30 seconds. The derivatization reaction proceeds for approximately two minutes at ambient temperature. The derivatized analyte is dried, reconstituted in 20 µL water and further diluted prior to mass spectral analysis. Chlorosulfonylacetyl chloride is also a useful reagent for derivatization of 2D gel isolates, but a modified synthetic procedure provides more consistent product yields. The modified procedure is discussed in Example 14.

### Example 3

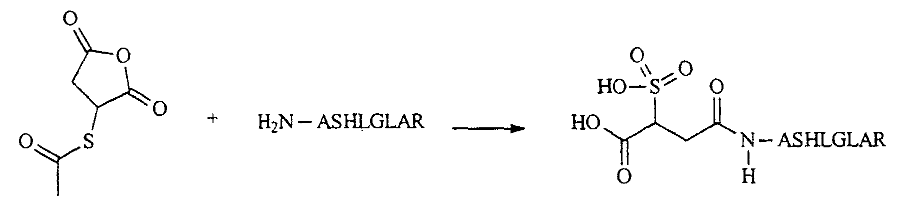

S-acetylmercaptosuccinic anhydride (commercially available from Aldrich Chemical Co., Milwaukee, WI) is prepared at a concentration of 0.1 M in dry tetrahydrofuran prior to use. ASHLGLAR (1 nmol, commercially available from Sigma Chemical Co., St. Louis MO) (SEQ ID NO: 1) is diluted into 20 µL of 0.05 M trimethylamine. The S-acetylmercaptosuccinic anhydride solution (5 µL) is added and the reaction mixture is vortexed for 30 seconds. The reaction proceeds for about two minutes at room temperature and is then oxidized with 10 µL of formic acid (88%):H2O2 (30%) prepared at a ratio of 19:1 (v:v). Oxidation proceeds for 16 hours at room temperature, and the sample is dried prior to dilution and mass spectral analysis. The concentration of the acidic moiety reagent is decreased by a factor of as much as 100 when derivatizing smaller quantities.

### Example 4

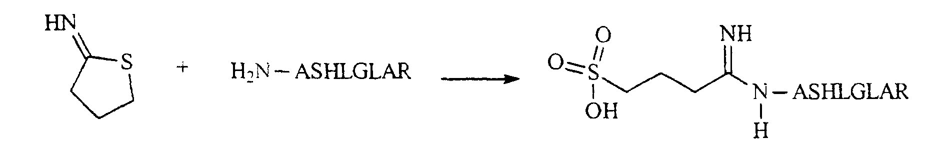

To ASHLGLAR (1 nmol. commercially available from Sigma Chemical Co., St. Louis. MO) (SEQ ID NO: 1) in 0.1 M trimethylamine (20 µL) is added 2-iminothiolane (Traut's reagent, commercially available from Aldrich Chemical Co., Milwaukee, WI) (3 µL, 0.1 M in deionized water). The reaction proceeds for 5 minutes at room temperature. The product is oxidized with 3 µL of formic acid (88%):H2O2 (30%) prepared at a ratio of 19:1 (v:v). Oxidation proceeds for 5 minutes at room temperature. and the derivatized analyte is dried prior to mass spectral analysis.

### Example 5

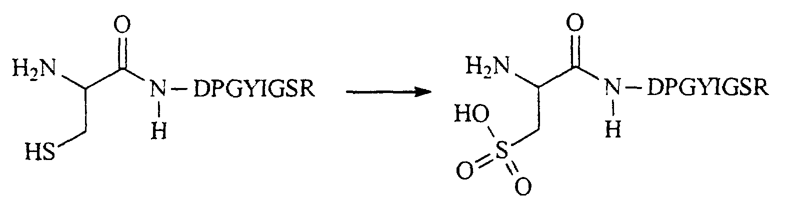

The polypeptide CDPGYIGSR (commercially available from Sigma Chemical Co., St. Louis, MO) (SEQ ID NO: 2) is oxidized by mixing 1 to 5 nM of polypeptide (in 5 - 20 µL water) with 10 µL of formic acid (88%):H2O2 (30%) prepared at a ratio of 19:1 (v:v). Oxidation proceeds for 30 minutes at room temperature. and the derivatized analyte is dried prior to mass spectral analysis.

### Example 6

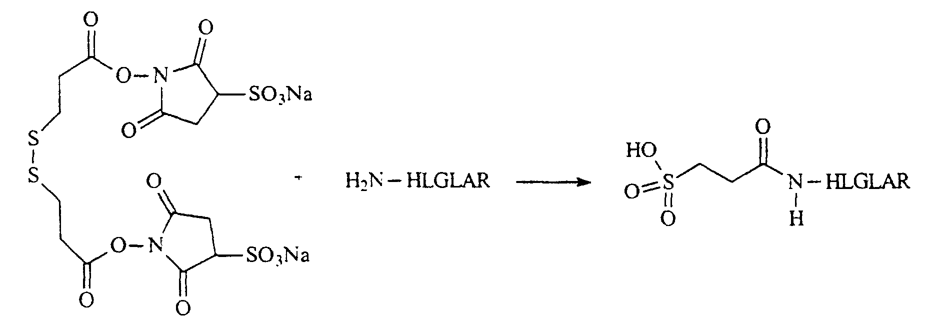

Dithiobis(sulfosuccinimidylpropionate) (DTSSP) (commercially available from Pierce Chemical Co., Rockford, IL) is taken up in phosphate-buffered saline at 50 nM / 20 µL and the pH is adjusted to 7.7 with 1 N NaOH. The solution (20 µL) is added to 1 µL of peptide HLGLAR (at 1 nM / µL) (SEQ ID NO: 3) and allowed to react for 30 minutes. The reaction is quenched with tris-hydroxymethyl-aminomethane (0.1M, 20 µL). The sample is desalted and oxidized with 10 µL of formic acid (88%):H2O2 (30%) prepared at a ratio of 19:1 (v:v). Oxidation proceeds for 30 minutes at room temperature, and the derivatized analyte is dried prior to mass spectral analysis.

### Example 7

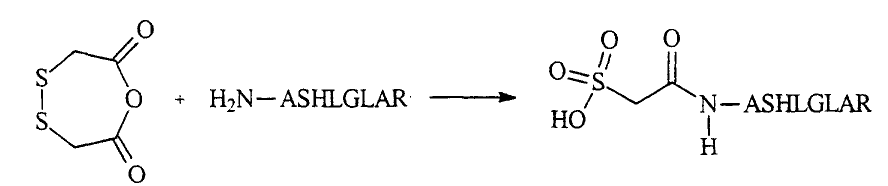

Dithiodiglycolic acid (0.93 g. 5.1 mmol, commercially available from Sigma Chemical Co.. St. Louis, MO) (alternatively, a polymer thereof of anhydride thereof (cyclic or acyclic) may be used) is dissolved in dichloromethane (20 mL) and placed under inert atmosphere. Dicyclohexylcarbodiimide (1.05 g, 5.1 mmol) is added in one portion. After about 96 hours, the precipitate is removed by filtration and the filtrate is concentrated in vacuo. The resulting material is taken up in diethylether and filtered. Again, the filtrate is concentrated in vacuo to provide dithiodiglycolic anhydride.

Dithiodiglycolic anhydride (the cyclic form is shown in this example) is prepared at a concentration of 0.1 M in dry tetrahydrofuran prior to use. ASHLGLAR (1 nmol) (SEQ ID NO: 1) is diluted into 20 µL of 0.05 M trimethylamine. The dithiodiglycolic anhydride solution (5 µL) is added and the reaction mixture is vortexed for 30 seconds. The reaction proceeds for about two minutes at room temperature. The resulting product is oxidized with 2 µL of formic acid (88%):H2O2 (30%) prepared at a ratio of 19:1 (v:v). Oxidation proceeds for 30 minutes at room temperature, and the derivatized analyte is dried prior to mass spectral analysis. The concentration of the acidic moiety reagent is decreased by a factor of as much as 100 when derivatizing smaller quantities.

### Example 8

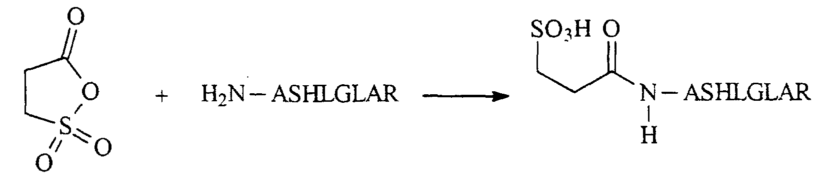

3-Sulfopropionic anhydride is prepared at a concentration of 0.1 M in dry tetrahydrofuran prior to use. ASHLGLAR (1 nmol) (SEQ ID NO: 1) is diluted into 20 µL of tetrahydrofuran:diisopropylethylamine 4:1 (v:v). The 3-sulfopropionic anhydride solution (2 µL) is added and the reaction mixture is vortexed for 30 seconds. The reaction proceeds for about two minutes at room temperature prior to dilution and mass spectral analyses. The concentration of the acidic moiety reagent is decreased by a factor of as much as 100 when derivatizing smaller quantities.

### Example 9

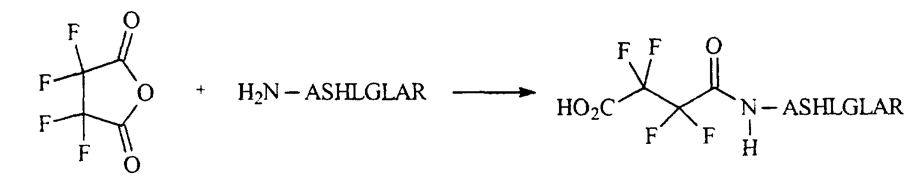

Tetrafluorosuccinic anhydride is prepared at a concentration of 0.1 M in dry tetrahydrofuran prior to use. ASHLGLAR (1 nmol) (SEQ ID NO: 1) is diluted into 20 µL of 0.05 M trimethylamine. The tetrafluorosuccinic anhydride solution (2 µL) is added and the reaction mixture is vortexed for 30 seconds. The reaction proceeds approximately two minutes at room temperature prior to dilution and mass spectral analysis. The concentration of the coupling reagent is decreased by a factor of approximately 100 when derivatizing smaller quantities.

### Example 10

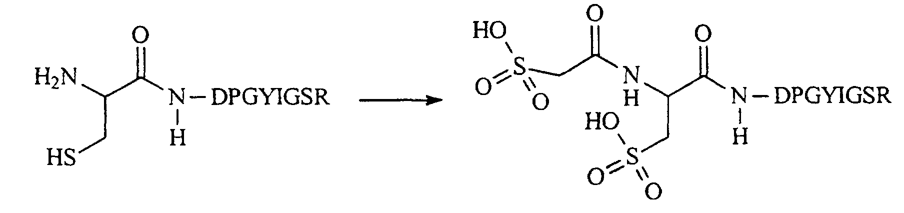

The polypeptide CDPGYIGSR (commercially available from Sigma Chemical Company, St. Louis, MO)(SEQ ID NO: 2) is mixed with 2 µL of 0.02M sulfoacetic acid, which is formed by mixing 2 µL of neat chlorosulfonylacetyl chloride (commercially available from Aldrich Chemical Co., Milwaukee, WI) with 500 µL of water. The mixture is dried and reconstituted in 20 µL of tetrahydrofuran:diisopropylethyl amine (4:1 v/v). 0.1 M chlorosulfonylacetyl chloride (2 µL) in dry tetrahydrofuran is added and the mixture is vortexed for 30 sec. The derivatization reaction proceeds for about 2 min. at ambient temperature. The derivatized analyte is dried and reconstituted in 10 µL of water. To that solution is added 10 µL of formic acid (88%):H2O2(30%) prepared at a ratio of 19:1 (v:v). Oxidation proceeds for 5 min. at room temperature, producing the derivatized peptide having two sulfonic acid groups near the N-terminus.

### Analysis Using a Mass Spectrometric Technique

Upon derivatization, the polypeptide or one or more peptides of the polypeptide are analyzed using a mass spectrometric technique. While the technique utilized is not limited, the preferred techniques are post-source decay (PSD) matrix-assisted laser desorption ionization (MALDI) and electrospray ionization tandem mass spectrometry. See, e.g., Spengler et al., "Peptide Sequencing by Matrix-assisted Laser-desorption Mass Spectrometry", Rapid Communications in Mass Spectrometry, Vol. 6, pp. 105 - 108 (1992); Spengler et al., "Fundamental Aspects of Postsource Decay in Matrix-Assisted Laser Desorption Mass Spectrometry", Journal of Physical Chemistry, Vol. 96, pp. 9678 - 9684 (1992); Kaufmann et al., "Mass Spectrometric Sequencing of Linear Peptides by Product-ion Analysis in a Reflectron Time-of-flight Mass Spectrometer Using Matrix-assisted Laser Desorption Ionization", Rapid Communications in Mass Spectrometry. Vol. 7, pp. 902 - 910 (1993); Kaufmann et al., "Sequencing of Peptides in a Time-of-flight Mass Spectrometer: Evaluation of Postsource Decay Following Matrix-assisted Laser Desorption Ionization (MALDI), International Journal of Mass Spectrometry and Ion Processes, Vol. 131, pp. 355 - 385 (1994); Kaufmann et al., "Post-source Decay and Delayed Extraction in Matrix-assisted Laser Desorption/Ionization-Reflectron Time-of-Flight Mass Spectrometry", Rapid Communications in Mass Spectrometry, Vol. 10, pp. 1199-1208 (1996); and Spengler, "Post-source Decay Analysis in Matrix-assisted Laser Desorption/Ionization Mass Spectrometry of Biomolecules", Journal of Mass Spectrometry, Vol. 32, pp. 1019 - 1036 (1997); Carr et al., "Integration of Mass Spectrometry in Analytical Biotechnology", Analytical Chemistry, Vol. 63, pp. 2802 - 2824, (1991); Yates III et al., "Mining Genomes With MS", Analytical Chemistry, Vol. 68, pp. 534A - 540A, (1996); Morris et al., "High Sensitivity Collisionally Activated Decomposition Tandem Mass Spectrometry on a Novel Quadrupole/Orthagonal Acceleration Time-of-Flight Mass Spectrometer", Rapid Communications in Mass Spectrometry, Vol. 10, 889 - 896, (1996). Most preferably, the techniques utilized are positive ion mode PSD MALDI and electrospray ionization tandem mass spectrometry. For convenience, Examples 11 and 12 below illustrate mass spectrometric techniques which may be utilized to analyze the polypeptide or peptides thereof.

As the present inventors have surprisingly discovered, appropriately derivatized peptides or peptides of the polypeptide provide MSMS spectra predominantly characterized by y-ions. As is well-known in the art, y-ions indicate ionized fragments containing the original C-terminus of the polypeptide or peptide. As used herein, the term "y-ion" also includes (y-NH3) ions; to illustrate, incomplete digestion products containing a second basic (for example) residue often yield abundant (y-NH3) ions. Preferably, the spectra produced by this method are substantially free of a-ions and b-ions. A-ions and b-ions are formed by cleavages on either side of backbone carbonyl groups. Importantly, charge is retained with the N-terminal fragment with a-ions and b-ions. As used herein, the term "substantially free of" with reference to a-ions and / or b-ions means that compared to the dominant y-ion series, a-ions and b-ions have a collective relative abundance of less than about 20%, preferably less than about 10%, and most preferably less than about 5%.

According to the method of the present invention, it is not necessary to analyze and / or identify all digestion products to identify the polypeptide thereof, particularly if the sequence of the polypeptide resides in a sequence database.

### Example 11 - PSD MALDI Sequencing Technique

In this example, the mass spectrometric technique is carried out on a Voyager DE-RP or Voyager DE-STR (PerSeptive Biosystems Inc., Framingham, MA) (or a suitable equivalent) equipped with a N2 laser (337 nm. 3 nsec pulse width, 20Hz repetition rate). The mass spectra are acquired in the reflectron mode with delayed extraction. External mass calibration is performed with a low-mass peptide standard, and mass measurement accuracy is typically ± 0.3 Da. The derivatized polypeptide or peptides are diluted to about 10 pM/µL in 0.1% trifluoroacetic acid (TFA). The samples are then diluted five-fold to ten-fold further in a-cyano-4-hydroxycinnamic acid (alphaCN) which is prepared by dissolving 10 mg in 1 mL of aqueous 50% acetonitrile containing 0.1% TFA. See, e.g., Beavis et al., "a-Cyano-4-hydroxycinnamic Acid as a Matrix for Matrix-assisted Laser Desorption Mass Spectrometry", Organic Mass Spectrometry, Vol. 27, pp. 156 - 158 (1992). Gel isolates are analyzed from thin film surfaces of a-cyano-4-hydroxycinnamic acid / nitrocellulose (alphaCN/NC) prepared by the fast evaporation method. See, e.g., Amott et al., "An Integrated Approach to Proteome Analysis: Identification of Proteins Associated with Cardiac Hypertrophy", Analytical Biochemistry, Vol. 258, pp. 1 - 18 (1998).

PSD MALDI tandem mass spectra are acquired for the derivatized analyte after isolation of the appropriate precursor ion using timed ion selection. The derivatized analytes can be analyzed using a number of MALDI matrices including, but not limited to alphaCN. alphaCN/NC and 2,5-dihydroxybenzoic acid (DHB). Fragment ions are refocused onto the final detector by stepping the voltage applied to the reflectron. Typical voltage ratios which may be used are as follows: 1.0000 (precursor ion segment), 0.9126, 0.6049, 0.4125, 0.2738, 0.1975, and 0.1213 (fragment segments). The individual segments are combined (or "stitched together" as is commonly used in the art) using software commercially available from PerSeptive Biosystems, Framingham, MA ("PSD" is selected from the analysis window). All precursor ion segments are acquired at low laser power (variable attenuator = 1450) for < 256 laser pulses to avoid saturating the detector. The laser power is increased (variable attenuator = 1650) for all of the remaining segments of the PSD MALDI acquisitions. Typically, 256 laser pulses are acquired for each fragment ion segment. The data are acquired at a digitization rate of 20 MHz.

### Example 12- An Electrospray Ionization Tandem Mass Spectrometry Sequencing Technique

In this example, the mass spectra are acquired using a capillary LC system (Perkin Elmer Biosystems, Foster City, CA) coupled to a LCQ ion trap mass spectrometer (ThermoQuest, San Jose, CA) equipped with a home-built microelectrospray source (uES). A 0.5 x 150 mm C18 LC column (Perkin Elmer Biosystems, Foster city, CA) is used with a flow rate of 5 µl/min. The LC mobile phases are water and acetonitrile, each containing 0.02% TFA. A typical gradient is 15% acetonitrile for 5 min., then 15-60% acetonitrile over 40 min. Flow rates of 0.5 µl/min to the uES source and 4.5 µl/min to a UV detector are achieved by placing a splitting tee (1/16", 0.25 mm bore, Valco, Houston, TX) after the LC column. The derivatized peptide or polypeptide samples are injected onto the LC column with an autosampler (ALCOTT, model 719, Norcross. GA). The uES source is housed on an X,Y,Z micrometer (New Focus, Inc., Santa Clara, CA) mounted to the front end of the instrument. The microelectrospray needle is a PicoTip (FS360-50-15-D) from New Objective (Cambridge, MA).

The electrospray tandem mass spectra are acquired using the following instrumental conditions: spray needle voltage 1.5 kV, heated capillary temperature 200 °C, and collision energy 35 eV, A mass range of 300-2000 m/z is used in each full-MS scan. The electrospray tandem mass spectra are acquired using data-dependent scanning in the "triple-play" mode which consists of three sequential microscans: 1) a full MS scan, 2) a zoom on selected ions to determine charge states, and 3) a MS/MS scans on appropriate ions selected from the zoom scans. These three scan events are repeated throughout the LC run.

### Interpreting the Fragmentation Pattern

The fragmentation pattern produced by the mass spectrometric analysis is interpreted to sequence the polypeptide. An artisan ordinarily skilled in the field of mass spectrometry will be able to manually interpret the fragmentation patterns of small polypeptides de novo without the aid of commercially available software or sequence databases. Similarly, the artisan will also be capable of sequencing the peptides of the polypeptides (the digest products) de novo. Alternatively, the artisan may use known aids for interpretation including, for example, commercially available software or sequence databases.

For example, sequences of the polypeptide or peptides thereof are efficiently and accurately determined with the y-ion fragmentation pattern produced via this invention. Identification of individual amino acid residues can be accomplished de novo by measuring mass differences between adjacent members in the y-ion series. Identification is then accomplished by comparing the measured mass differences to the known amino acid residue masses (see Table I herein above). For example, a measured mass difference of 71.1 Da corresponds to alanine. The reading direction is also established directly from the mass spectrum. The direction is from the C-terminus to the N-terminus if measuring from low-mass to high-mass. The reading direction is from the N-terminus to the C-terminus if measuring from high-mass to low-mass.

The sequences of the polypeptide, and peptides thereof, may also be efficiently and accurately determined using software which accepts mass spectral fragmentation data, either uninterpreted y-ion series masses or sequence tags derived from the y-ion masses, as inputs for sequence database searches. Such search software commonly utilized by the skilled artisan include, but are not limited to, "Protein Prospector" (commercially available from the University of California at San Francisco or http://prospector.ucsf.edu) and "Peptide Search" (commercially available from the European Molecular Biology Laboratory at Heidelberg, Germany or http://www.mann.embl-heidelberg.de).

The fragmentation pattern produced by this invention can be searched against a number of sequence databases including, but not limited to, the NCBI non-redundant database (ncbi.nlm.mh.gov/blast/db.nr.z), SWISPROT (ncbi.nlm.gov/repository/SWISS-PROT/sprot33.dat.z), EMBL (FTP://ftp.ebi.ac.uk/pub/databases/peptidesearch/), OWL (ncbi.nlm.nih.gov/repository/owl/FASTA.z), dbEST (ncbi.nlm.nih.gov/repository/dbESTIdbEST.weekly.fasta.mmddyy.z) and Genebank (ncbi.nlm.nih.gov/genebank/genpept.fsa.z). The entire sequence of the polypeptide of interest can often be retrieved from the sequence database by searching the fragmentation data produced from one or more of the relevant peptide derivatives formed using the methods of this invention.

Of course, when using database searching techniques, it is most efficient to limit the searches by specifying that only y-ions or (y-NH3) ions are allowed fragments because y- and (y-NH3) ions are the most prominent species observed in the fragmentation patterns wherein the present methods are utilized. Other fragment ion types like a-, b-, (b+H2O), (b-H2O), (b-NH3) and internal cleavage ions can be disallowed because they are not prominent in the spectra of the peptides derivatized using the methods of the present invention. The derivatives formed with the present invention provide simple fragmentation patterns that often yield greater database search specificity than can be obtained from the spectra of the same peptides without derivatization.

The methods of the present invention are further illustrated in the non-limiting examples below. In these examples, the ordinarily skilled artisan will recognize that numbers of "candidate proteins" produced may differ from those disclosed herein as new proteins are continually added to databases.

### Example 13

PSD MALDI tandem mass spectrometry of high-mass polypeptides is demonstrated with oxidized insulin B-chain FVNQHLC(SO₃H)GSHLVEALYLVC(SO₃H)GERGFFYTPKA (SEQ ID NO: 4) (MMcalc = 3495.9 Da) (commercially available from Sigma Chemical Co., St. Louis, MO). The mass of this polypeptide far exceeds the upper limit of most conventional triple quadrupole instruments. The tandem mass spectrum of the native polypeptide shows a relatively intense ion series consisting substantially of y-ions. All y-ions between y9 and y24 are readily observed. The sequence-specific fragments defining the N-terminus of the molecule, y25 to y29, are absent from the spectrum.

The spectrum of this polypeptide is improved by derivatization using the acidic moiety reagent sulfobenzoic acid cyclic anhydride (commercially available from Aldrich Chemical Co., St. Louis, MO). The derivatized polypeptide shows significant enhancement of the y-ions derived from the N-terminal region of the molecule (y25, y26, y27, y28, and y29). A complete series of y-ions is observed from y8 to y29 following derivatization. No prominent b-ions are detected.

### Example 14

A polypeptide separated by two-dimensional gel electrophoresis is in-gel digested with trypsin to produce peptides of the polypeptide. The peptides show several intense MH+ signals by MALDI including ions at m/z 1060.8, 1090.8, 1271.0, 1299.0, 1312.0, 1344.0, 1399.1, 1450.1, 1460.1, and 1794.4. Database searching using the Protein Prospector Software against the entries in the NCBI protein sequence library produces a list of forty-one candidate proteins that match five or more of the input tryptic masses (search parameters: MW range 1,000 to 150,000, isoelectric point from 3.0 to 10.0, oxidized Met allowed as a side-reaction and a conservative mass tolerance of +/- 0.6 Da).

The peptides of the polypeptide can be derivatized with any one of several of the reagents discussed in the previous examples. They include, but are not limited to, 2-sulfobenzoic acid cyclic anhydride, 3-sulfopropinoic anhydride or chlorosulfonylacetyl chloride. In this example, chlorosulfonyl chloride is used as the acidic moiety reagent. The derivatization procedure for low-level peptides isolated from 2D gel electrophoresis is modified to improve reproducibility and derivative yields. Typically, the peptide extract from the 2D gel is concentrated to near dryness (5 to 10 µL) on a speed vac. The concentrates are acidified with 15 µL of 0.1% TFA and cleaned up using commercially available C18 mini-columns (ZipTip™, Millipore Corporation. Bedford, MA 01730). The cleaned up sample is dried on a speed vac and reconstituted in 10 µL of base (THF:DIEA 19:1 v/v). In this example, 2 µL of a chlorosulfonylacetyl chloride solution (2 µL of the neat liquid in 1 mL THF) is added and the reaction proceeds for 1 to 2 min at room temperature. The derivatized samples are again dried on a speed vac and reconstituted in 10 µL of 0.1% TFA prior to analysis. At this stage, the sample can be mixed with MALDI matrix and a portion loaded onto the sample stage for analysis, or it may be cleaned up again using a commercially available C18 mini-column (ZipTip™, Millipore Corporation). The cleaned up sample can then be eluted directly from the ZipTip onto the MALDI sample stage in a 1 - 2 µL volume of acetonitrile:0.1 % TFA (1:1 v/v) containing 10 mg/mL of MALDI matrix. This latter procedure allows loading all of the recovered derivatives onto the MALDI sample stage for analysis thereby improving overall sensitivity of the method. PSD MALDI tandem mass spectrometry is carried out on the derivatized peptide weighing about 1572 Da. A sequence tag is obtained having y-ions at m/z 574.5. 661.7, 875.9, 1003.8, 1103.9, 1204.6. 1304.1, and the (MH+ - derivative) ion at m/z 1451.0. The spectrum is searched against the protein sequence database, and six candidate proteins (all mitochondrial aspartate aminotransferases) are returned (search parameters: MW range 1,000 to 150,000, parent ion mass tolerance +/- 0.6 Da, fragment ion mass tolerance +/- 2.5 Da, Par(mi)Frag(av), allowed number of missed ions = 1). This level of specificity is obtained because the database search is constrained to consider y-ions as the only allowed fragments. This search constraint is possible because N-terminal and internal cleavage ions are not prominent in the tandem mass spectra of these derivatives (due to derivatization according to the present invention). Much less specificity is obtained (239 candidate proteins returned) when this same tandem mass spectrum is searched against the full library and all fragment ion types (a, b, (b + H2O), (b - NH3), (b - H2O), internal and y) are allowed. The peptide was identified as FVTVQTISGTGALR (SEQ ID NO: 5).

Confirmation of the protein identification is sought by PSD MALDI of the derivative weighing about 1916 Da. The spectrum contains 7 fragment ions at m/z 724.6, 1154.2, 1299.3, 1439.0, 1551.9, 1665.5, and 1779.2, in addition to the protonated precursor ion. This spectrum is searched against the database assuming the fragments to be y-ions. The search does not return mitochondrial aspartate aminotransferase or any other candidate protein. Researching the database allowing both y and (y - NH3) ions returns 16 candidate proteins, 10 of which are mitochondrial aspartate aminotransferases. This latter search confirms the protein identification. Inclusion of the (y - NH3) ions as possible fragments is required for this particular search because the peptide is an incomplete tryptic product (ILIRPLYSNPPLNGAR) (SEQ ID NO: 6) that contains a second arginine residue. As stated herein above, derivatized incomplete digestion products often exhibit (y - NH3) fragment ions in the PSD tandem mass spectra.

### Example 15

An in-gel tryptic digest of a protein isolated from 2D gel electrophoresis is analyzed by LC electrospray tandem mass spectrometry on an LCQ ion trap following derivatization according to Example 14 herein. All spectra are acquired unattended in an automated data-dependent mode using the "triple play" sequence of microscans. The tandem mass spectrum of a derivatized tryptic peptide (MH+ = 971.5) shows several y-type product ions including m/z 401.1, 538.3, 651.3. 750.4. The measured y-ions are used as inputs, along with the MH+ mass of the underivatized tryptic peptide (971.5 - 122 = 849.5), for database searching. Database searching using the Protein Prospector Software against the NCBI protein sequence library returns 3 candidate proteins (search parameters: MW range all, parent ion tolerance +/- 0.6 Da, fragment ion tolerance +/- 1.0 Da, monoisotopic parent and fragment ions, and no missed ions allowed). All three of the candidate proteins returned from the database search are haptoglobins. The peptide was identified as VVLHPER (SEQ ID NO: 7).

Confirmation of the protein identification is sought by searching the database using the tandem mass spectrum produced from a second derivatized peptide (MH+ of derivative = 771.4 Da). The spectrum showed several ions including y-type ions at m/z 322.1, 436.2 and 535.3. Using the same search parameters as above, three peptide sequences match the input data from the electrospray tandem mass spectrum. One of the three peptide sequences corresponds to that of haptoglobin. The identification of that peptide was established as NVNFR (SEQ ID NO: 8). This result confirms the identity of the protein.

### Example 16

The method of the present invention is readily utilized to identify variant polypeptides. The MALDI mass spectrum of a tryptic digest of an enzyme isolate shows many tryptic masses identical to those of the commercial protease Savinase® (commercially available from Novo Nordisk. Copenhagen, Denmark). One of the expected tryptic peptides GVLVVAASGNSGAGSISYPAR (MH+ = 1933 Da) (SEQ ID NO: 9) is missing from the spectrum, and an unknown ion is observed at m/z 1963. This suggests that the isolate is a Savinase® variant. Eleven different single amino acid changes could account for the observed +30 Da mass shift (4 G → S, 4 A → T, and 3 V → E). The PSD MALDI tandem mass spectrum is obtained following derivatization such as illustrated in Example 2 herein. A complete series of y-ions is observed for the 21 amino acid peptide. The spectrum verifies that the glycine at residue 14 is converted to a serine. The masses of all the y-ions are measured in this spectrum. The spectrum is automatically interpreted using the peptide ladder sequencing program. which is included in the PerSeptive Biosystems MALDI data system.

### Example 17

The use of disulfonic acid derivatives, with both sulfonic acid groups near the N-terminus of the peptide, is demonstrated by comparison of the MALDI PSD spectra produced from CDPGYIGSR (SEQ ID NO: 2) derivatized according to Example 5 and according to Example 10 herein. The derivative formed by performic acid oxidation of the sulfhydryl group of the cysteine residue (Example 5) is analyzed using PSD MALDI from a matrix of alpha-cyano-4-hydroxycinnamic acid. The resulting PSD spectrum is dominated by the y7 fragment ion formed by cleavage of the labile Asp-Pro amide bond. Lower mass y-type ions show relatively low abundance. For example, the abundances of the y3 and y4 ions relative to the y7 ion are only about 8% and 5% respectively (peak height ratios). Without intending to be limited by theory, it is believed that the "mobile" ionizing proton is preferentially localized on the basic Asp-Pro amide nitrogen atom because the Pro amide nitrogen is more basic than the other backbone amide groups. This charge localization is thought to yield preferential fragmentation of the amide bond between Asp and protonated Pro. This problem is minimized by addition of a second sulfonic acid group near the N-terminus of the peptide according to Example 10. Addition of the second sulfonic acid group requires addition of a second "mobile" proton to produce the positively charged ion used for MALDI PSD analysis. Again, without intending to be limited by theory, this second proton is believed to be free to ionize other backbone amide groups because the relatively basic Pro group is already ionized. Protonation of other backbone amide groups leads to enhanced fragmentation of those groups and increased relative abundance of the corresponding y-ions in the MALDI PSD spectrum. In the PSD spectrum of the derivative formed according to Example 10, the abundances of the y3 and y4 ions increase to about 41% and 57% respectively (peak height ratios) relative to that of the y7 ion.

### Kits of the Present Invention

A further embodiment of the present invention are kits which may be utilized to determine the amino acid sequence of a polypeptide. The kits comprise:
(a) one or more acidic moiety reagents providing acidic moieties having pKas less than about 2 when coupled with the polypeptide or one or more peptides of the polypeptide; and
(b) means for derivatizing the N-terminus of the polypeptide or the N-termini of one or more peptides of the polypeptide with one or more acidic moiety reagents.

The kits of the present invention may be adapted to a mass spectrometer in a similar fashion as the sample holder described in. for example, Patterson, U.S. Patent No. 5,827,659, assigned to PerSeptive Biosystems, Inc., issued October 27, 1998.

Optionally, the kits may further comprise one or more verification peptides to test. for example, the accuracy of the mass spectrometric technique. Reference mass spectral data may also be optionally be included.

The acidic moiety reagents which may be included in the kits are described above.

Especially preferred means for derivatizing include those which allow convenient derivatization by the analyst or any other person interested in obtaining the derivatized polypeptide or peptides. A particularly preferred means for derivatizing comprises one or more containment devices to contain, for example, the acidic moiety reagent and ultimately the polypeptide / peptides of interest.

Suitable containment devices include, for example, vials, tubes, pipette tips, plates, sample holders, and multi-well plates. Optional convenience is provided wherein the acidic moiety reagent resides within the containment device so that it need not be added by the analyst. For example, the acidic moiety reagent may reside on the inside of a pipette tip and activated as the polypeptide or peptides are pulled into the tip with a suitable buffer. The containment device is most preferably disposable, but need not be.

The acidic moiety reagent may also be bound to a solid support. For example, the reagent may be support-bound inside a pipette tip. The polypeptide or peptides of interest may be taken up in an appropriate buffer system and repeatedly drawn over the bound reagent. After reaction, an appropriate quantity of the derivatized polypeptide or peptides may be loaded directly onto the MALDI mass spectrometry sample stage, or injected into an electrospray ionization mass spectrometry device.

One or more buffer systems used to facilitate derivatization may also be included in the kits of the present invention. The buffer system appropriate for inclusion is dependent upon the acidic moiety reagent included. Examples of preferred buffer systems are disclosed in the derivatization examples above. Particularly preferred buffer systems include, but are not limited to, tertiary amine solutions (both aqueous and non-aqueous (for example, solutions in tetrahydrofuran)) and neat tertiary amines. Particularly preferred tertiary amines include trimethylamine, triethylamine, and diisopropylethylamine.

The kits of the present invention may also comprise one of more digestion aids such as those described herein above. Digestion aids may be chemical or enzymatic. For example, trypsin, endoproteinase Lys C, endoproteinase Arg C, and / or chymotrypsin, preferably, trypsin. endoproteinase Lys C, and / or endoproteinase Arg C, and most preferably trypsin may be included as a digestion aid. Chemical digestion aids, such as cyanogen bromide. may also be included herein.

## Claims

1. A method of determining the amino acid sequence of a polypeptide **characterized in that** it comprises:
(a) derivatizing the N-terminus of the polypeptide or the N-termini of one or more peptides of the polypeptide with one or more acidic moieties having pKas of less than 2, when coupled with the polypeptide or peptides, to provide one or more derivatized analytes;
(b) analyzing one or more derivatized analytes using a mass spectrometric technique to provide a fragmentation pattern, and
(c) interpreting the fragmentation pattern.

2. A method according to Claim 1 **characterized in that** the mass spectrometric technique is MALDI PSD mass spectrometry, or electrospray ionization tandem mass spectrometry.

3. A method according to Claim 1 or 2 **characterized in that** interpretation of the fragmentation pattern comprises using a commercially available software program or database.

4. A method according to any of Claims 1-3 **characterized in that** the polypeptide is a synthetic polypeptide.

5. A method according to any of Claims 1-4 **characterized in that** the peptides of the polypeptide are produced by digestion.

6. A method according to Claim 5 **characterized in that** the digestion is enzymatic digestion.

7. A method according to any of Claims 1-6 **characterized in that** the acidic moiety is one or more sulfonic acids or a disulfonic acid derivative.

8. A kit for use in determining the amino acid sequence of a polypeptide **characterized in that** it comprises:
(a) one or more acidic moiety reagents providing one or more acidic moieties having pKas of less than 2 when coupled with the polypeptide or one or more peptides of the polypeptide; and
(b) means for derivatizing the N-terminus of the polypeptide or the N-termini of one or more peptides of the polypeptide with one or more acidic moiety reagents.

9. A kit according to Claim 8 **characterized in that** the means for derivatizing comprises one or more containment devices.

10. A kit according to Claim 8 or 9 **characterized in that** it further comprises one or more digestion aids.

## Patentansprüche

1. Verfahren zur Bestimmung der Aminosäuresequenz eines Polypeptids, **dadurch gekennzeichnet, dass** es umfasst:
(a) Derivatisieren des N-Terminus des Polypeptids oder der N-Termini eines oder mehrerer Peptide des Polypeptids mit einer oder mehreren sauren Einheiten mit pKas von weniger als 2 bei Kupplung mit dem Polypeptid oder den Peptiden, um einen oder mehrere derivatisierte Analyte vorzusehen;
(b) Analysieren eines oder mehrerer derivatisierter Analyte unter Verwendung einer Massenspektrometrietechnik, um ein Fragmentationsmuster vorzusehen; und
(c) Interpretieren des Fragmentationsmusters.

2. Verfahren nach Anspruch 1, **dadurch gekennzeichnet, dass** die Massenspektrometrietechnik MALDI PSD-Massenspektrometrie oder Elektrospray-Ionisations-Tandem-Massenspektrometrie ist.

3. Verfahren nach Anspruch 1 oder 2, **dadurch gekennzeichnet, dass** die Interpretation des Fragmentationsmusters die Verwendung eines kommerziell erhältlichen Softwareprogramms oder einer Datenbank umfasst.

4. Verfahren nach mindestens einem der Ansprüche 1-3, **dadurch gekennzeichnet, dass** das Polypeptid ein synthetisches Polypeptid ist.

5. Verfahren nach mindestens einem der Ansprüche 1-4, **dadurch gekennzeichnet, dass** die Peptide des Polypeptids durch Digestion erzeugt werden.

6. Verfahren nach Anspruch 5, **dadurch gekennzeichnet, dass** die Digestion eine enzymatische Digestion ist.

7. Verfahren nach mindestens einem der Ansprüche 1-6, **dadurch gekennzeichnet, dass** die saure Einheit eine oder mehrere Sulfonsäuren oder ein Disulfonsäurederivat ist.

8. Kit zur Verwendung bei der Bestimmung der Aminosäuresequenz eines Polypeptids, **dadurch gekennzeichnet, dass** es umfasst:
(a) ein oder mehrere Reagentien mit sauren Einheiten, welche eine oder mehrere saure Einheiten mit pKas von weniger als 2 vorsehen bei Kupplung mit dem Polypeptid oder einem oder mehreren Peptiden des Polypeptids; und
(b) Mittel zur Derivatisierung des N-Terminus des Polypeptids oder der N-Termini eines oder mehrerer Peptide des Polypeptids mit einem oder mehreren Reagentien mit sauren Einheiten.

9. Kit nach Anspruch 8, **dadurch gekennzeichnet, dass** das Mittel zum Derivatisieren ein oder mehrere Behältnisvorrichtungen umfasst.

10. Kit nach Anspruch 8 oder 9, **dadurch gekennzeichnet, dass** es weiterhin eine oder mehrere Disgestionshilfen umfasst.

## Revendications

1. Procédé pour déterminer la séquence d'acides aminés d'un polypeptide, **caractérisé en ce qu'**il consiste :
(a) à dériver le N-terminal du polypeptide ou les N-terminaux d'un ou plusieurs peptides du polypeptide avec un ou plusieurs fragments acides ayant un pKa inférieur à 2 lorsqu'il est couplé avec le peptide ou les peptides pour fournir un ou plusieurs analytes dérivés ;
(b) à analyser un ou plusieurs analytes dérivés en utilisant une technique de spectrométrie de masse pour fournir un schéma de fragmentation, et
(c) à interpréter le schéma de fragmentation.

2. Procédé selon la revendication 1, **caractérisé en ce que** la technique de spectrométrie de masse est une spectrométrie de masse MALDI PSD ou une spectrométrie de masse en tandem électroprojection-ionisation.

3. Procédé selon la revendication 1 ou 2, **caractérisé en ce que** l'interprétation du schéma de fragmentation comprend l'utilisation d'un programme de logiciel disponible dans le commerce ou d'une base de données.

4. Procédé selon l'une quelconque des revendications 1 à 3, **caractérisé en ce que** le polypeptide est un polypeptide synthétique.

5. Procédé selon l'une quelconque des revendications 1 à 4, **caractérisé en ce que** les peptides du polypeptide sont produits par digestion.

6. Procédé selon la revendication 5, **caractérisé en ce que** la digestion est une digestion enzymatique.

7. Procédé selon l'une quelconque des revendications 1 à 6, **caractérisé en ce que** le fragment acide est un ou plusieurs acides sulfoniques ou un dérivé d'acide disulfonique.

8. Nécessaire destiné à être utilisé dans la détermination de la séquence d'acides aminés d'un polypeptide, **caractérisé en ce qu'**il comprend:
(a) un ou plusieurs réactifs de fragment acide fournissant un ou plusieurs fragments acides ayant un pKa inférieur à 2 lors du couplage avec le polypeptide ou un ou plusieurs peptides du polypeptide ; et
(b) un moyen pour dériver le N-terminal du polypeptide ou les N-terminaux d'un ou plusieurs peptides du polypeptide avec un ou plusieurs réactifs de fragment acide.

9. Nécessaire selon la revendication 8, **caractérisé en ce que** le moyen pour dériver comprend un ou plusieurs dispositifs de retenue.

10. Nécessaire selon la revendication 8 ou 9, **caractérisé en ce qu'**il comprend, en outre, un ou plusieurs auxiliaires de digestion.
